(19) Europäisches Patentamt
European Patent Office
Office européen des brevets

(11) Veröffentlichungsnummer: **0 373 388 A2**

# (12) EUROPÄISCHE PATENTANMELDUNG

(21) Anmeldenummer: **89121486.8**

(51) Int. Cl.5: **C07D 499/88, A61K 31/43**

(22) Anmeldetag: **21.11.89**

(30) Priorität: **26.11.88 DE 3839987**

(43) Veröffentlichungstag der Anmeldung:
**20.06.90 Patentblatt 90/25**

(84) Benannte Vertragsstaaten:
**AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Lattrell, Rudolf, Dr.**
**Heuhohlweg 6h**
**D-6240 Königstein/Taunus(DE)**
Erfinder: **Dürckheimer, Walter, Dr.**
**Im Lerchenfeld 45**
**D-6234 Hattersheim am Main(DE)**
Erfinder: **Seibert, Gerhard, Prof. Dr.**
**Gläserweg 21**
**D-6100 Darmstadt(DE)**

## (54) Penemderivate und Verfahren zu ihrer Herstellung.

(57) Penemderivate der Formel

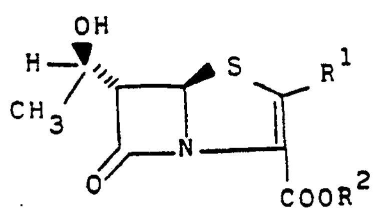

mit $R^1$ gleich 3-Oxocyclobutyl und $R^2$ = H oder einer esterbildenden Gruppe, gegen bakterielle Infektionen wirksame pharmazeutische Präparate, die solche Penemderivate enthalten, Verfahren zur Herstellung der Penemderivate und der pharmazeutischen Präparate und Verwendung der Penemderivate zur Bekämpfung bakterieller Infektionen werden beschrieben.

EP 0 373 388 A2

# Penemderivate und Verfahren zu ihrer Herstellung

Die Erfindung betrifft Penemderivate, die in 2-Stellung des Penemrings durch einen cycloaliphatischen Rest substituiert sind und die eine sehr gute antimikrobielle Wirkung gegen gram-positive und gram-negative Bakterien besitzen und deshalb als Arzneimittel zur Behandlung von mikrobiellen Infektionen geeignet sind, sowie Verfahren zu ihrer Herstellung.

Penem-Derivate und ihre antibakterielle Wirksamkeit sind bekannt, z.B. aus den Patentschriften US 4 386 030, EP 2210 oder EP 278 911. Diese Substanzen sind jedoch noch nicht in jeder Hinsicht befriedigend.

Die Erfindung betrifft daher Penemderivate der Formel I

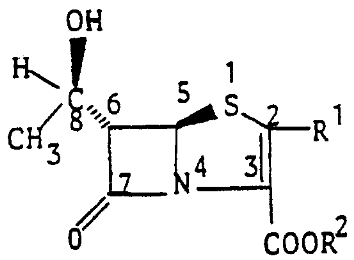

I

und deren pharmazeutisch verträgliche Salze,
worin
$R^1$ $C_3$-$C_6$-Oxocycloalkyl,
$C_3$-$C_6$-(1,1-bis-$C_1$-$C_3$-Alkyloxy)cycloalkyl,
$C_3$-$C_6$-(1,1-$C_2$-$C_3$-Alkylendioxy)cycloalkyl,
$C_3$-$C_6$-($C_1$-$C_3$-Alkylimino)cycloalkyl,
$C_3$-$C_6$-Aryliminocycloalkyl (mit Aryl gleich Phenyl, Thienyl oder Furyl),
$C_3$-$C_6$-Hydroxyiminocycloalkyl
$C_3$-$C_6$-($C_1$-$C_3$-Alkyloxyimino)cycloalkyl,
in denen der Cycloalkylrest unsubstituiert oder ein-oder zweifach durch $C_1$-$C_3$-Alkyl, vorzugsweise Methyl, $C_1$-$C_3$-Alkyloxy, vorzugsweise Methoxy, durch Halogen, vorzugsweise Chlor oder durch Methylen substituiert ist,
$R^2$ Wasserstoff
$C_1$-$C_5$-Alkanoyloxy-$C_1$-$C_3$-alkyl,
$C_1$-$C_5$-Alkoxycarbonyloxy-$C_1$-$C_3$-alkyl,
5-Methyl-1,3-dioxolen-2-on-4-yl-methyl, Phthalidyl bedeutet,
und in denen die bevorzugte Stereochemie in Stellung 5 = R, in Stellung 6 = S und in Stellung 8 = R ist.

Als besonders bevorzugt kommen beispielsweise die folgenden Gruppen in Betracht:
$R^1$ 3-Oxocyclobutyl, funktionelle Derivate der Ketogruppe, wie

Acetale 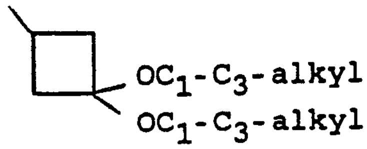

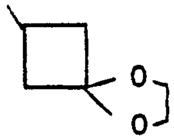 oder 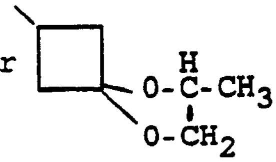

Schiffbasen 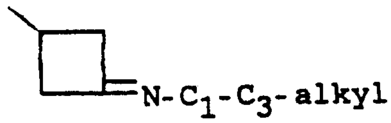 oder 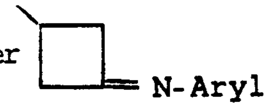

Oxime 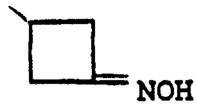 oder 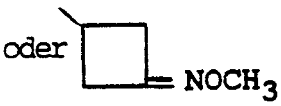

worin n = 0-3 ist, und Aryl Phenyl, Thienyl, Furyl bedeutet,

$R^2$ Wasserstoff

Esterreste, die in vivo unter Bildung der freien Carbonsäure abgespalten werden können, z.B. Acetoxymethyl, Propionyloxymethyl, Isopropionyloxymethyl, N-Butyryloxymethyl, Isobutyryloxymethyl, Pivaloyloxymethyl, Isovaleryloxymethyl, 1-Acetoxyethyl, 1-Acetoxypropyl, 1-Pivaloyloxyethyl, 1-Methoxycarbonyloxyethyl, 1-Ethoxycarbonyloxyethyl, 1-Isopropoxycarbonyloxyethyl; Methoxycarbonyloxymethyl; Phthalidyl, 5-Methyl-1,3-dioxolen-2-on-4-yl-methyl.

Die Erfindung umfaßt weiterhin Verfahren zur Herstellung von Verbindungen der Formel I und ihrer pharmazeutisch verträglichen Salze, die dadurch gekennzeichnet sind, daß man

a) eine Verbindung der Formel II

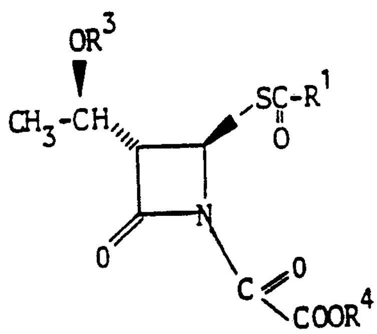

II,

worin $R^1$ die zu Formel I genannte Bedeutung hat, $R^3$ Wasserstoff oder eine Hydroxyschutzgruppe, $R^4$ eine Carboxyschutzgruppe bedeutet, mit Trialkylphosphiten umsetzt,

α) eine gegebenenfalls vorhandene Schutzgruppe $R^3$ und $R^4$ abspaltet und

ß) falls erforderlich, das erhaltene Produkt in ein pharmazeutisch verträgliches Salz überführt, oder

b) eine Verbindung der Formel III

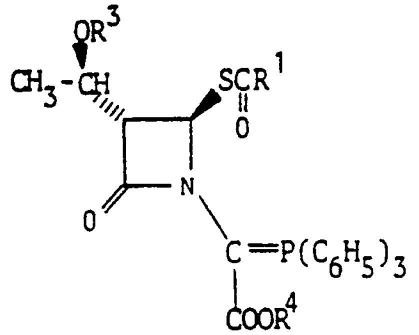

III,

worin $R^1$, $R^3$ und $R^4$ die zu Formel II genannte Bedeutung haben, durch Erhitzen cyclisiert,

α) eine gegebenenfalls vorhandene Schutzgruppe abspaltet und

ß) falls erforderlich, das erhaltene Produkt in ein pharmazeutisch verträgliches Salz überführt und

c) falls funktionelle Derivate von Ketoverbindungen erhalten werden sollen, die nach den Verfahrensvarianten a) oder b) erhaltenen Verbindungen der Formel I mit einer Ketogruppe im Rest $R^1$ in an sich bekannter Weise zum Beispiel in Acetale, Schiffbasen oder Oxime überführt, und

d) falls in vivo spaltbare Ester erhalten werden sollen, die nach den Verfahrensvarianten a), b) oder c) erhaltenen Verbindungen der Formel I, in der $R^2$ für Wasserstoff steht, in an sich bekannter Weise in die unter $R^2$ angegebenen Ester überführt.

Nach dem Verfahren a) werden die Verbindungen der Formel II unter der Einwirkung von 2-4 Äquivalenten Trialkylphosphit, vorzugsweise Trimethylphosphit oder Triethylphosphit, cyclisiert. Die Reaktion wird ausgeführt bei Temperaturen zwischen 60°C und 200°C, vorzugsweise zwischen 90°C und 150°C in einem inerten aprotischen Lösungsmittel. Geeignet sind z.B. chlorierte Kohlenwasserstoffe wie 1,1,2-Trichlorethan, aromatische Kohlenwasserstoffe wie Benzol, Toluol oder Xylol. Die Reaktionsdauer hängt von den Reaktanten, der Temperatur und dem Lösungsmittel ab und liegt normalerweise zwischen 1/2 und etwa 72 Stunden. Nach diesem Verfahren werden die Verbindungen der Formel I zunächst in geschützter Form erhalten.

Die Schutzgruppen werden in an sich bekannter Weise abgespalten. Bevorzugte Schutzgruppen $R^3$ für die 8-Hydroxygruppe sind z.B. die Trimethylsilyl-, die Tetrahydropyranyl- oder 2-Methoxyprop-2-yl-Gruppe, die durch Säurehydrolyse, oder die tert.-Butyldimethylsilyl-Gruppe, die mittels Tetrabutylammoniumfluorid abgespalten wird.

Geeignete Schutzgruppen $R^4$ für die Carboxygruppe sind insbesondere die hydrogenolytisch abspaltbare p-Nitrobenzylgruppe, die Allyl- oder 2-Chlorallylgruppe, die mit $Pd[P(C_6H_5)_3]_4$ oder die Trimethylsilylethylgruppe, die mittels $Bu_4NF$ abgespalten wird.

Die Ausgangsverbindungen der Formel II werden nach literaturbekannten Verfahren erhalten.

Nach dem Verfahren b) wird eine Verbindung der Formel III unter Bildung einer geschützten Verbindung der Formel I cyclisiert. Die Reaktion verläuft vorzugsweise in einem inerten aprotischen Lösungsmittel wie Toluol, Xylol, Dioxan, Tetrahydrofuran, Diethoxyethan bei Temperaturen zwischen Zimmertemperatur und Rückflußtemperatur der Reaktionsmischung.

Nach der Cyclisierungsreaktion werden die Reaktionsprodukte wie üblich durch Säulenchromatographie oder Kristallisieren gereinigt. Die Schutzgruppen werden anschließend wie oben beschrieben, entfernt. Die Ausgangsverbindungen der Formel III werden ebenfalls nach literaturbekannten Verfahren erhalten.

Sollen nach Verfahren d) Verbindungen der Formel I erhalten werden, in der $R^2$ für $C_1$-$C_5$-Alkanoyloxy-$C_1$-$C_3$-alkyl, $C_1$-$C_5$-Alkoxycarbonyloxy-$C_1$-$C_3$-alkyl, Phthalidyl oder 5-Methyl-1,3-dioxolen-2-on-4-ylmethyl steht, so setzt man in an sich bekannter Weise die Verbindungen der Formel I, in denen $R^2$ = Wasserstoff bedeutet,
mit einer Verbindung der Formel IV

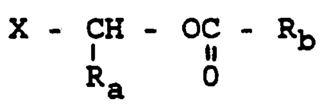

IV,

worin $R_a$ = Wasserstoff oder eine $C_1$-$C_2$-Alkylgruppe, $R_b$ = eine $C_1$-$C_5$-Alkyl- oder eine $C_1$-$C_5$-Alkyloxygruppe bedeutet, oder mit V oder VI

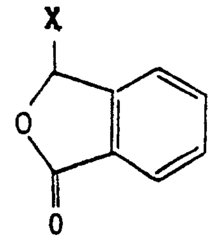

(V)

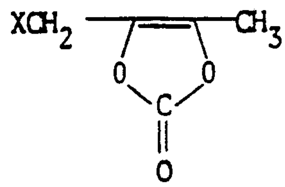

(VI)

worin X für Halogen, vorzugsweise Chlor, Brom oder Jod steht, um.

Es werden Verfahren angewendet, wie sie für Veresterungsreaktionen bekannt sind.

Als pharmazeutisch verträgliche Salze der Verbindungen der Formel I seien beispielsweise erwähnt Lithium-, Natrium-, Kalium-, Calcium-, Magnesiumsalze oder Salze mit organischen Aminen wie Diethylamin, Benethamin, Piperazin, Tromethamin.

Die erfindungsgemäß erhaltenen Verbindungen der Formel I und ihre pharmazeutisch verträglichen Salze zeigen bemerkenswert gute antibakterielle Wirksamkeit sowohl gegen grampositive als auch gramnegative bakterielle Keime.

Auch gegen penicillinase- und cephalosporinase-bildende Bakterien sind die Verbindungen der Formel I unerwartet gut wirksam. Da sie zudem günstige toxikologische und pharmakologische Eigenschaften zeigen, stellen sie wertvolle Chemotherapeutika dar.

Die Erfindung betrifft somit auch Arzneipräparate zur Behandlung von mikrobiellen Infektionen, die durch einen Gehalt an einer oder mehreren der erfindungsgemäßen Verbindungen charakterisiert sind.

Die erfindungsgemäßen Produkte können auch in Kombinationen mit anderen Wirkstoffen, beispielsweise aus der Reihe der Penicilline, Cephalosporine oder Aminoglykoside zur Anwendung kommen.

Die Verbindungen der Formel I und ihre pharmazeutisch verträglichen Salze können oral, intramuskulär oder intravenös verabfolgt werden.

Arzneipräparate, die eine oder mehrere Verbindungen der Formel I als Wirkstoff enthalten, können hergestellt werden, indem man die Verbindungen der Formel I mit mehreren pharmakologisch verträglichen Trägerstoffen oder Verdünnungsmittel, wie Füllstoffen, Emulgatoren, Gleitstoffen, Geschmackskorrigentien, Farbstoffen oder Puffersubstanzen vermischt in eine geeignete galenische Zubereitungsform bringt, wie Tabletten, Dragees, Kapseln oder eine zur parenteralen Applikation geeignete Suspension oder Lösung.

Als Träger- oder Verdünnungsmittel seien beispielsweise erwähnt Traganth, Milchzucker, Talkum, Agar-Agar, Polyglykole, Ethanol und Wasser. Puffersubstanzen sind beispielsweise organische Verbindungen, wie z.B. N,N'-Dibenzylethylendiamin, Diethanolamin, Ethylendiamin, N-Methylglucamin, N-benzylphenethylamin, Diethylamin, Tris-(hydroxymethyl)aminomethan (Tromethamin), oder anorganische Verbindungen wie Phosphatpuffer, Natriumbicarbonat, Natriumcarbonat. Für die parenterale Applikation kommen vorzugsweise Suspensionen oder Lösungen in Wasser mit oder ohne Puffersubstanzen in Betracht. Es ist auch möglich, die Wirkstoffe als solche ohne Träger- oder Verdünnungsmittel in geeigneter Form, beispielsweise in Kapseln, zu applizieren.

Geeignete Dosen der Verbindungen der Formel I oder ihrer pharmazeutisch verträglichen Salze liegen bei etwa 0,4 g, vorzugsweise 0,5 g pro Tag bis maximal etwa 20 g, vorzugsweise 4 g/Tag für einen Erwachsenen von etwa 75 kg Körpergewicht.

Es können Einzel- oder im allgemeinen Mehrfachdosen verabreicht werden, wobei die Einzeldosen den Wirkstoff in einer Menge von etwa 50 bis 1000 mg, vorzugsweise von etwa 100 bis 500 mg, enthalten kann.

Die folgenden Ausführungsbeispiele für erfindungsgemäß herstellbare 5R, 6S-Verbindungen dienen zur weiteren Erläuterung der Erfindung.

**Beispiel 1:**

(5R,6S)-6-[(1R)-Hydroxyethyl]-2-(3-oxocyclobutyl)-penem-3-carbonsäure

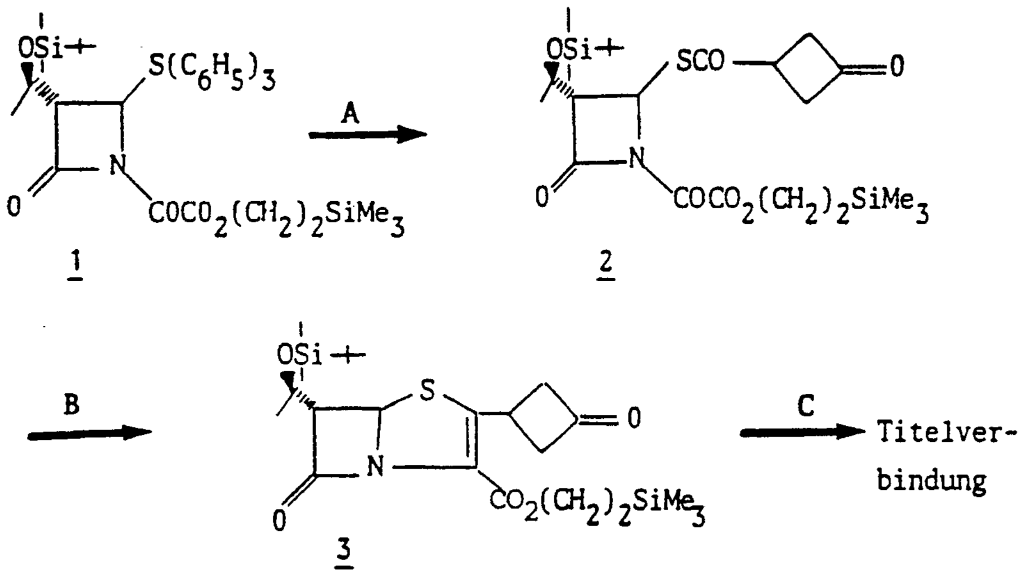


1 2 3

Stufe A:

Zur Lösung von 2.04 g (3 mmol) 1 in 12 ml Acetonitril wird bei 0° C unter Argon während 5 Minuten eine Lösung von 562 mg (3.3 mmol) Silbernitrat, 260 mg (3.3 mmol) Pyridin und 100 mg (3.12 mmol) Methanol in 6 ml Acetonitril zugegeben. Es wird 1 Stunde im Eisbad gerührt, das Acetonitril im Vakuum abdestilliert und der Rückstand in 40 ml Methylenchlorid gelöst. Die Lösung wird zweimal mit je 15 ml Wasser gewaschen, über Magnesiumsulfat getrocknet, filtriert und auf ein Volumen von 20 ml eingeengt. Zu dieser Lösung des Silbersalzes wird bei 0° C eine Lösung von 510 mg (3.8 mmol) 3-Oxocyclobutancarbonsäurechlorid in 5 ml Methylenchlorid gegeben. Es wird 2 Stunden im Eisbad und 30 Minuten bei Zimmertemperatur gerührt, das ausgefallene Silberchlorid wird abgesaugt und mit Methylenchlorid gewaschen. Die organische Phase wird zweimal mit je 15 ml Wasser gewaschen und getrocknet. Nach Abdestillieren des Lösungsmittels wird der Rückstand über Kieselgel, das mit 10 % Wasser desaktiviert wurde, mit Cyclohexan : Essigester (5 : 1) chromatographiert. Man erhält 1.05 g 2 (66 %) als farbloses dickflüssiges Öl.
$^1$H-NMR ($CDCl_3$): $\delta$= 0.06 (s, 9H), 0.09 (s, 6H), 0.86 (s, 9H), 1.12 (m, 2H, $CH_2Si$), 1.26 (d, J= 6Hz, 3H, $CHCH_3$), 3.2-3.6 (m, 5 Cyclobutan-H), 3.49 (dd, 3-H), 4.38 (m, 3H, $CO_2CH_2$ und CH-$CH_3$), 6.01 (d, J= 2.5Hz, 4-H).

Stufe B:

Eine Lösung von 530 mg (1 mmol) 2 in 25 ml trockenem Toluol wird zum Sieden erhitzt und sodann eine Lösung von 644 mg (4 mmol) Triethylphosphit in 2.5 ml Toluol zugegeben. Es wird weitere 5 Stunden unter Rückfluß erhitzt, gekühlt und mit 5 ml 1N $KHSO_4$, sodann fünfmal mit je 25 ml Wasser gewaschen. Nach dem Trocknen ($MgSO_4$) wird das Lösungsmittel i.Vak. entfernt und der Rückstand über Kieselgel desaktiviert und 10 % Wasser, mit Toluol : Essigester (10:1) chromatographiert. Nach dem Einengen der Produktfraktionen erhält man 375 mg (75 %) 3 als farblosen kristallinen Feststoff.
$^1$H-NMR ($CDCl_3$) $\delta$= 0.06 (s, 9H), 0.10 (s, 6H), 0.88 (s, 9H), 1.08 (m, 2H, $CH_2Si$), 1.28 (d, J= 6Hz, 3H, $CHCH_3$), 3.15-3.55 (m, 4 Cyclobutan-H), 3.70 (dd, J= 5 und 1Hz, 6-H), 4.25 (m, 3H, $CO_2CH_2$ und $CHCH_3$), 4.52 (m, 1 Cyclobutan-H), 5.58 (d, J= 1Hz, 5-H).

Stufe C: Herstellung der Titelverbindung

Zur Lösung von 318 mg (0.6 mmol) 3 in 1.2 ml Tetrahydrofuran werden 3 ml (3 mmol) einer 1N $Bu_4NF$-Lösung in Tetrahydrofuran zugegeben. Nach 10 Minuten bei Zimmertemperatur werden 12 ml (3.6 mmol) 0.3 N Essigsäure zugegeben und die Lösung i.Vak. auf 10 ml eingeengt. Die wäßrige Lösung wird über

Adsorberharz XAD-2 (2 x 25 cm Säule) charomatographiert, Elutionsmittel: Wasser, gefolgt von Wasser:Isopropanol (9:1). Die Produktfraktionen werden gefriergetrocknet und das erhaltene Tetrabutylammoniumsalz der Titelverbindung (120 mg) über Reversephase-Kieselgel RP 18 mit Waser:Isopropanol (9:1) rechromatographiert. Nach Gefriertrocknen der Produktfraktionen erhält man 39 mg (23 %) eines amorphen farblosen Feststoffs.

$^1$H-NMR (DMSO-$d_6$) δ= 1.16 (d, J= 6Hz, $CHCH_3$), 2.94-3.18 (m, 2 Cyclobutan-H), 3.20-3.45 (m, 2 Cyclobutan-H), 3.56 (dd, J= 5 und 1Hz, 6-H), 3.93 (m, 1H, $CHCH_3$), 4.72 (m, 1 Cyclobutan-H), 5.62 (bs, OH), 5.50 (d, J= 1Hz, 5-H).

**Beispiel 2**

(5R,6S)-6-[(1R)-Hydroxyethyl]-2-(3-oxocyclobutyl)-penem-3-carbonsäure-kaliumsalz

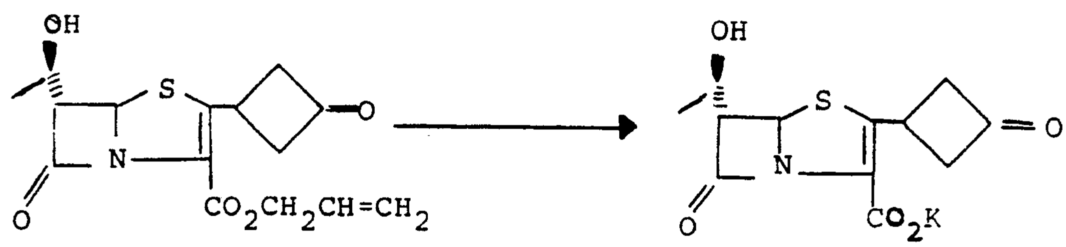

Zur Lösung von 8.1 g (25 mmol) 1 - diese Ausgangsverbindung vom Schmp. 121-123 °C wurde aus der Verbindung 1 des Beispiels 3 in Analogie zu der dort angegebenen Reaktionsfolge A → B→ C unter Verwendung von 3-Oxocyclobutancarbonsäurechlorid hergestellt - in 80 ml Methylenchlorid und 30 ml Ethylacetat wird bei Zimmertemperatur unter Argon eine Lösung von 4.57 g (25 mmol) 2-Ethylenhexansäure-Kaliumsalz in 50 ml Ethylacetat und sodann 1.97 (7.5 mmol) Triphenylphosphin und 1.27 (1.1 mmol) Tetrakis-(triphenylphosphin)palladium(0) zugegeben. Die Mischung wird 4 Stunden bei Zimmertemperatur gerührt, mit 300 ml Methylenchlorid verdünnt und zweimal mit je 250 ml Wasser extrahiert. Die wäßrige Phase wird dreimal mit je 100 ml Methylenchlorid gewaschen und gefriergetrocknet. Es verbleiben 7.5 g braunes amorphes Produkt. Es wird in 20 ml Wasser gelöst und mit 100 ml Aceton verdünnt. Nach kurzem Reiben tritt Kristallisation des Kaliumsalzes ein. Nach 4 Stunden Kühlen im Eisbad wird abgesaugt, mit Aceton gewaschen und über $P_2O_5$ getrocknet. Ausbeute: 4.7 g. Aus der eingeengten Mutterlauge werden weitere 0.7 g isoliert.

Gesamtausbeute: 5.4 g (67,3 % d.Th.)

$^1$H-NMR (DMSO-$d_6$) δ= 1.16 (d, J= .6Hz, 3H, $CHCH_3$), 2.90-3.38 (m, 4-Cyclobutan-H), 3.45 (dd, J= 5 und 1Hz, 6-H), 3.90 (m, 1H, $CHCH_3$), 4.88 (m, 1 Cyclobutan-H), 5.21 (d, J= 4Hz, OH), 5.45 (d, J= 1Hz, 5-H)

**Beispiel 3**

5R,6S-[(1R)-Hydroxyethyl]-2-(3,3-dimethoxycyclobutyl)-penem-3-carbonsäure-Natriumsalz

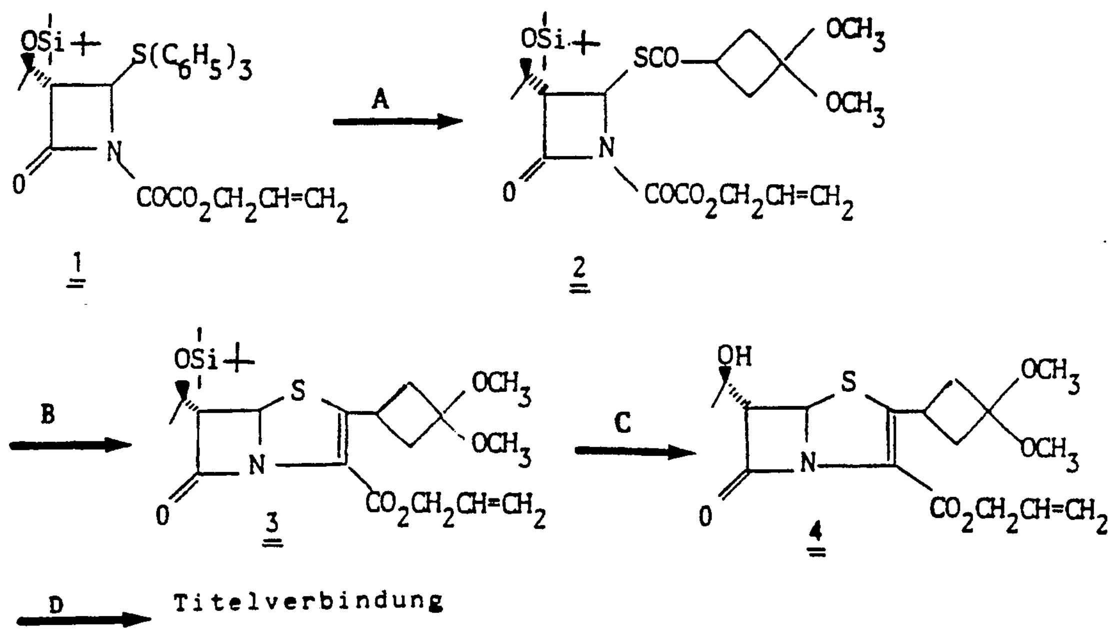


Zur Lösung von 1.85 g (3 mmol) 1 in 12 ml Acetonitril wird bei 0° C unter Argon während 5 Minuten eine Lösung von 562 mg (3.3 mmol) Silbernitrat, 260 mg (3.3 mmol) Pyridin und 100 mg (3.12 mmol) Methanol in 6 ml Acetonitril zugegeben. Es wird 1 Stunde im Eisbad gerührt, das Lösungsmittel im Vakuum abdestilliert und der Rückstand in 40 ml Methylenchlorid gelöst. Die Lösung wird zweimal mit je 15 ml Wasser gewaschen, über $MgSO_4$ getrocknet und auf 20 ml eingeengt. Zur Lösung des Silbersalzes wird bei 0° C eine Lösung von 4 mmol rohem 3,3-Dimethoxycyclobutancarbonsäurechlorid - hergestellt aus 4 mmol Natriumsalz und 4 mmol Oxalylchlorid in 5 ml Methylenchlorid 30 Minuten 5° C bis Zimmertemperatur - in Methylenchlorid gegeben. Nach 2 Stunden Rühren im Eisbad und 30 Minuten bei Zimmertemperatur wird das ausgefallene Silberchlorid abgesaugt, mit Methylenchlorid gewaschen und die organische Phase nacheinander mit 20 ml 1N Natriumbicarbonatlösung und zweimal mit je 15 ml Wasser gewaschen. Nach dem Trocknen ($MgSO_4$) wird der Rückstand der organischen Phase über Kieselgel desaktiviert mit 10 % Wasser, mit Cyclohexan : Essigester (5:1, dann 2:1) chromatographiert. Die Produktfraktionen ergeben 1.09 g 2 als dickflüssiges Öl (70 %).
$^1$H-NMR ($CDCl_3$): $\delta$= 0.10 (s, 6H), 0.85 (s, 9H), 1.22 (d, J= 6Hz, 3H, $CHCH_3$), 2.35-2.55 (m, 4 Cyclobutan-H), 3.12 und 3.15 (je 1s, 2 x 3H, $OCH_3$), 3.47 (dd, 3-H), 4.36 (m, 1H, $CHCH_3$), 4.77 (d, 2H, $CO_2CH_2$), 5.28-5.45 (m, 2H, = $CH_2$), 5.86-6.03 (m, 1 = CH), 5.98 (d, J= 2.5Hz, 4-H).

Stufe B;

Eine Mischung aus 515 mg (1 mmol) 2 und 664 mg (4 mmol) Triethylphosphit in 27.5 ml Toluol werden 5 Stunden unter Rückfluß erhitzt. Das Lösungsmittel wird im Vakuum entfernt und der Rückstand über Kieselgel, desaktiviert mit 10 % Wasser, mit Toluol : Essigester (5:1) chromatographiert. Die Produktfraktionen ergeben 160 mg (33 %) 3 als farbloses Harz.
$^1$H-NMR ($CDCl_3$): $\delta$= 0.08 (s, 6H), 0.88 (s, 9H); 1.24 (d, J= 6Hz, 3H, $CHCH_3$), 2.10-2.68 (m, 4 Cyclobutan-H), 3.12 und 3.14 (je 1s, 2 x 3H, $OCH_3$), 3.65 (dd, J= 5 und 1Hz, 6-H), 3.9-4.1 (m, 1 Cyclobutan-H), 4.22 (m, 1H, $CHCH_3$), 4.62-4.72 (m, 2H, $CO_2CH_2$), 5.20-5.43 (m, 2H, =$CH_2$), 5.52 (d, J= 1Hz, 5-H), 5.85-6.00 (m, 1 =CH).

Stufe C:

230 mg (0.48 mmol) 3 werden in 6 ml Tetrahydrofuran gelöst, 300 mg (5 mmol) Essigsäure, gefolgt von 0.64 ml (0.64 mmol) einer 1N $Bu_4NF$-Lösung in Tetrahydrofuran werden zugegeben und die Lösung 65 Stunden bei 20° C belassen. Nach Zugabe von 30 ml Diethylether wird mit 10 ml 1N Natriumbicarbonatlösung sowie 3 x mit je 10 ml Wasser gewaschen, mit Magnesiumsulfat getrocknet und das Lösungsmittel im

Vakuum entfernt. 4 (120 mg, 68 %) wird als farbloses Harz erhalten.
$^1$H-NMR ($CDCl_3$): δ = 1.36 (d, J = 6Hz, 3H, $CHCH_3$), 2.10-2.70 (m, 4 Cyclobutan-H), 3.13 und 3.16 (je 1s, 2 x 3H, $OCH_3$), 3.61 (dd, J = 5 und 1Hz, 6-H), 3.9-4.1 (m, 1 Cyclobutan-H), 4.22 (m, 1H, $CHCH_3$) 4.60-4.81 (m, 2H, $CO_2CH_2$), 5.22-5.44 (m, 2H, $=CH_2$), 5.58 (d, J = 1Hz, 5-H), 5.87-6.02 (m, 1 =CH)

Stufe D: Herstellung der Titelverbindung

110mg (0.3 mmol 4 werden unter Argon in einem Gemisch aus 1 ml Methylenchlorid und 0.4 ml Ethylacetat gelöst. Sodann wird eine Lösung von 60 mg (0.36 mmol) 2-Ethylhexansäure-Natriumsalz in 0.6 ml Ethylacetat, gefolgt von 24 mg (0.09 mmol) Triphenylphosphin und 15 mg (0.013 mmol) Tetrakis-(triphenylphosphin)-palladium(0), zugegeben. Die Mischung wird 4 Stunden bei Zimmertemperatur gerührt. Anschließend werden 6 ml Wasser zugegeben. Die Wasserphase wird fünfmal mit je 10 ml Methylenchlorid gewaschen und gefriergetrocknet. Der Rückstand wird über XAD-2 mit Wasser : Isopropanol (10:1) chromatographiert. Man erhält 68 mg (65 %) Natriumsalz in Form eines gelblichen amorphen Feststoffs.
$^1$H-NMR (DMSO-$D_6$): δ = 1.13 (d, J = 6Hz, $CHCH_3$), 1.85-2.40 (m, 4 Cyclobutan-H), 3.03 und 3.06 (je 1s, 2 x 3H, $OCH_3$), 3.45 (dd, J = 5 und 1Hz, 6-H), 3.90 (m, 1H, $CHCH_3$), 4.40-4.55 (m, 1 Cyclobutan-H), 5.40 (d, J = 1Hz, 5-H).

**Beispiel 4**

(5R,6S)-6-[(1R)-Hydroxyethyl]-2-(3,3-ethylendioxycyclobutyl)-penem-3-carbonsäure-Natriumsalz

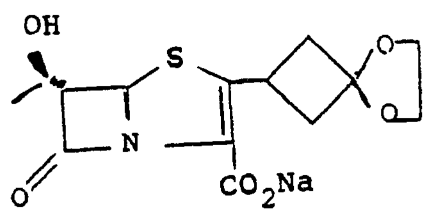

Die Verbindung wird in Analogie zu Beispiel 3 aus der dortigen Verbindung 1 unter Verwendung von 3,3-Ethylendioxycyclobutan-carbonsäurechlorid nach der Reaktionsfolge A → B → C → D hergestellt. Nach Chromatographie über XAD-2 wird das Natriumsalz als amorpher farbloser Feststoff erhalten.
$^1$H-NMR (DMSO-$d_6$) δ = 1.14 (d, J = 6Hz, $CHCH_3$), 2.1-2.45 (m, 4 Cyclobutan-H), 3.42 (dd, J = 5 und 1Hz, 6-H), 3.70-3.95 (m, 5H, $CHCH_3$ und Ethylen), 4.55 (m, 1 Cyclobutan-H), 5.25 (bs, OH), 5.40 (d, J = 1Hz, 5-H).

**Beispiel 5**

(5R,6S)-6-[(1R)-Hydroxyethyl]-2-(3-methoxyiminocyclobutyl)-penem-3-carbonsäure-Kaliumsalz

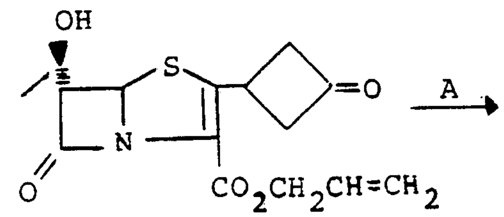


1

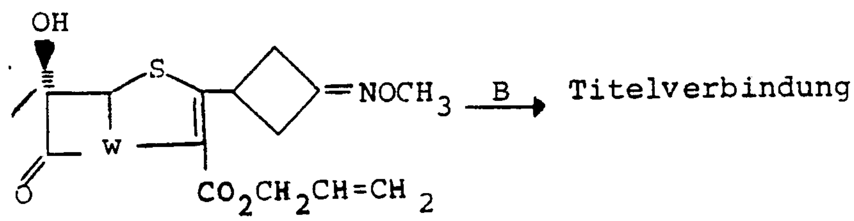


2

Stufe A:

194 mg (0.6 mmol) I werden in 3.6 ml Ethanol und 0.6 ml Methylenchlorid gelöst und eine Lösung von 1.2 mmol 0-Methylhydroxylamin in 2 ml Ethanol zugegeben. Nach 20 Stunden bei 5° C wird mit Eiswasser und Methylenchlorid aufgearbeitet und der Rückstand der organischen Phase über Kieselgel mit Toluol : Essigester (2:1) chromatographiert. Die Produktfraktionen ergeben 170 mg 2 (80 %) als farbloses Harz.
$^1$H-NMR ($CDCl_3$) $\delta$ = 1.36 (d, J = 6Hz, $CHCH_3$), 2.88-3.42 (m, 4 Cyclobutan-H), 3.72 (dd, J = 5 und 1Hz, 6-H), 3.85 (s, 3H, $OCH_3$), 4.23 (m, 1H, $CHCH_3$), 4.38 (m, 1 Cyclobutan-H), 4.62-4.82 (m, 2H, $CO_2CH_2$), 5.22-5.45 (m, 2H, $=CH_2$), 5.61 (d, J = 1Hz, 5-H), 5.88-6.04 (m, 1H, =CH).

Stufe B:

Herstellung der Titelverbindung

159 mg (0.45 mmol) 2 werden mit 82 mg 2-Ethylhexansäure-Kaliumsalz, 36 mg Triphenylphosphin und 23 mg Tetrakis-(triphenylphosphin)-palladium(0) in Analogie zu Beispiel 2 umgesetzt. Das nach Gefriertrocknung erhaltene Rohprodukt (104 mg) wird über XAD-2 (Elution mit Wasser : Isopropanol (10:1) gereinigt. Man erhält 60 mg (38 %) Kaliumsalz der Titelverbindung als farblosen amorphen Feststoff.
$^1$H-NMR ($D_2O$): $\delta$ = 1.31 (d, J = 6Hz, $CHCH_3$), 2.90-3.41 (m, 4 Cyclobutan-H), 3.82 (s, 3H, $OCH_3$), 3.88 (dd, J = 5 und 1Hz, 6-H), 4.22 (m, 1H, $CHCH_3$), 4.33-4.48 (m, 1 Cyclobutan-H), 5.63 (d, J = 1Hz, 5H).

**Beispiel 6**

(5R,6S)-6-[(1R)-Hydroxyethyl]-2-(3-hydroxyiminocyclobutyl) penem-3-carbonsäure-Kaliumsalz

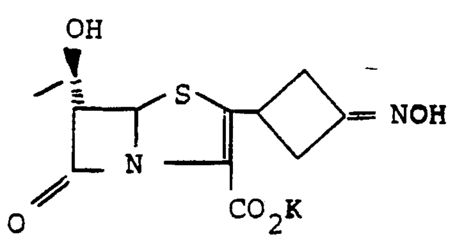

Die Verbindung wird in Analogie zu Beispiel 5 aus der dortigen Verbindung 1 durch Umsetzen mit Hydroxylamin in Ethanol und nachfolgender Abspaltung der Allylschutzgruppe hergestellt. Nach Chromatographie über XAD-2 wird das Kaliumsalz als farbloser amorpher Feststoff erhalten. $^1$H-NMR ($DMSO-d_6$) $\delta$=1.16 (d, J= 6Hz, $CHCH_3$), 2.6-3.2 (m, 4 Cyclobutan-H), 3.60 (dd, J= 5 und 1Hz, 6-H), 3.86-4.0 (m, 1H, $CHCH_3$), 4.5-4.7 (m, 1 Cyclobutan-H), 5.53 (d, J= 1Hz, 5-H).

**Beispiel 7**

(5R,6S)-6-[(1R)-Hydroxyethyl]-2-(3-oxocyclobutyl)-penem-3-carbonsäure-pivaloyloxymethylester

160 mg (0.5 mmol) Kaliumsalz der Verbindung aus Beispiel 2 werden in 1.5 ml Dimethylsulfoxid gelöst und 121 mg (0.5 mmol) Pivaloyloxymethyljodid zugegeben. Es wird 1 Stunde bei Zimmertemperatur belassen. Zur Lösung werden sodann 10 ml 2 % Natriumbicarbonatlösung zugegeben und 5 x mit je 5 ml Essigester extrahiert. Die organische Phase wird zweimal mit je 5 ml Wasser gewaschen und über $MgSO_4$ getrocknet. Der Rückstand der organischen Phase wird über Kieselgel, desaktiviert mit 10 % Wasser, mit Toluol : Essigster (2:1) chromatographiert. Die Produktfraktionen ergeben 149 mg (75 %) eines gelblichen Feststoffs
$^1$H-NMR ($CDCl_3$): $\delta$= 1.22 (s, 9H), 1.35 (d, J= 6Hz, $CHCH_3$), 3.15-3.58 (m, 4 Cyclobutan-H), 3.72 (dd, J= 5 und 1 Hz, 6-H), 4.25 (m, 1H, $CHCH_3$), 4.42 (m, 1 Cyclobutan-H), 5.62 (d, J= 1Hz, 5-H), 5.81 und 5.92 (AB, J= 6Hz, $CO_2CH_2$).

**Beispiel 8**

(5R,6S)-6-[(1R)-Hydroxyethyl]-2-(3-oxocyclobutyl)-penem-3-carbonsäure-(1-pivaloyloxy)ethylester.

Die Verbindung wird in Analogie zu Beispiel 7 aus dem Kaliumsalz des Beispiels 2 und (1-Pivaloyloxy)-ethyljodid hergestellt. Nach chromatographischer Reinigung wird ein 1:1-Isomerengemisch als amorpher gelber Feststoff in 20 % Ausbeute erhalten.
$^1$H-NMR ($CDCl_3$) : $\delta$= 1.18; 120 (je 1s, 9H), 1.38 (2 x d, 3H J= 6Hz, $CHCH_3$), 1.57 (2 x d, 3H, $CO_2CHCH_3$), 3.12-3.55 (m, 4 Cyclobutan-H), 3.72 (2 x dd, J= 5 und 1Hz, 6-H), 4.22 (m, 1H, $CHCH_3$), 4.43 (m, 1 Cyclobutan-H), 5.61 (2 x d, J= 1Hz, 5-H), 6.92 (m, 1Hz, 1H $CO_2CHCH_3$)

**Beispiel 9**

(5R,6S)-6-[(1R)-Hydroxyethyl]-2-(3-hydroxyiminocyclobutyl) penem-3-carbonsäure-pivaloyloxymethylester

Die Verbindung wird in Analogie zu Beispiel 7 aus dem Kaliumsalz des Beispiels 6 und Pivaloyloxymethyljodid hergestellt. Nach chromatographischer Reinigung wird ein amorpher gelber Feststoff erhalten.
$^1$H-NMR ($CDCl_3$): $\delta$= 1.22 (s, 9H), 1.36 (d,J= 6Hz, 3H, $CHCH_3$) 2.92-3.45 (m, 4 Cyclobutan-H), 3.72 (dd, J= 5 und 1Hz, 6-H), 4.22 (m, 1H, $CHCH_3$), 4.32 (m, 1 Cyclobutan-H), 5.61 (d, J= 1Hz, 5-H), 5.81 und 5.90 (AB, J= 6Hz, $CO_2CH_2$).

**Beispiel 10**

(5R,6S)-6-[(1R)-Hydroxyethyl]-2-(3-phenyliminocyclobutyl) penem-3-carbonsäure-tetrabutylammoniumsalz

3

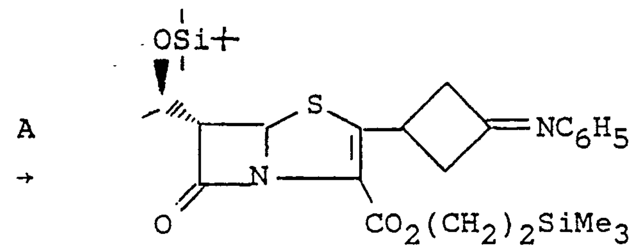


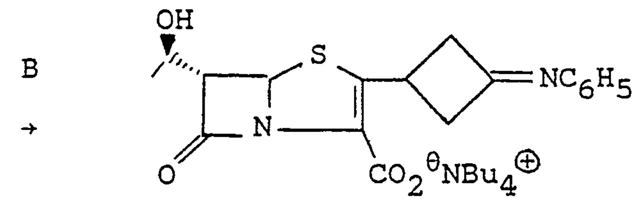


Stufe A:

Eine Lösung von 294 mg (0.5 mmol) 3 (Beispiel 1), 51 mg (0.55 mmol) Anilin und 5 mg p-Toluolsulfonsäure in 3 ml Toluol werden 1 Stunde unter Rückfluß erhitzt. Die Lösung wird über Kieselgel, desaktiviert mit 10 % Wasser, mit Toluol Essigester (20:1) chromatographiert. Nach dem Einengen der Produktfraktionen erhält man 250 mg (87 %) eines bräunlichen, amorphen Feststoffs.
$^1$H-NMR ($CDCl_3$): $\delta$ = 0.06 (s,9H), 0.09 (s, 6H), 0.90 (s, 9H), 1.05 (m, 2H, $CH_2Si$), 1.28 (d,J=6Hz, 3H, $CHCH_3$), 2.1-2.6 (m, 4 Cyclobutan-H), 3.62 (dd, J=5 und 1Hz, 6H), 4.0-4.3 (m, 4H, 1 Cyclobutan-H, $CO_2CH_2$ und $CHCH_3$), 5.58 (d, J=1Hz, 5-H); 6.45, 6.70, 7.05 (je 1, 2 und 2H, arom. H).

Stufe B:

Zur Lösung von 1.43 mg (0.25 mmol) der Verbindung aus Stufe A in 2.5 ml Tetrahydrofuran werden 1.25 ml (1.25 mmol) einer 1 N $Bu_4NF$-Lösung in Tetrahydrofuran gegeben. Nach 10 Minuten bei Zimmertemperatur werden 0.75 ml 2 N Essigsäure zugegeben, und die Lösung wird im Vakuum eingeengt. Der harzige Rückstand wird mit 5 ml Eiswasser digeriert, die wäßrige Lösung wird verworfen, das Harz in 2 ml Wasser:Isopropanol (4:1) gelöst. Diese Lösung wird über Adsorberharz XAD-2 (2x25 cm-Säule) mit Wasser:Isopropanol (4:1) chromatographiert. Die Fraktionen 5 - 8 (je 10 ml) ergeben nach Gefriertrocknung 40 mg der Titelverbindung als farblosen, amorphen Feststoff.
$^1$H-NMR ($D_2O$) : $\delta$ = 0.92 (m, 12H, $NBu_4$), 1.33 (m, 11H, 8H, $NBu_4$; 3H, $CHCH_3$), 1.62 (m, 8H, $NBu_4$), 2.2-2.7 (m, 4 Cyclobutan-H), 3.18 (m, 8H, $NBu_4$), 3.82 (dd, J=5 und 1 Hz, 6-H), 3.95 (m, 1H, $CHCH_3$), 4.22 (m, 1 Cyclobutan-H), 5.60 (d, J=1 Hz, 5 H); 6.58, 6.80, 7.12 (je 1, 2 und 2H, arom. H).

## Ansprüche

1. Penemderivate der Formel I

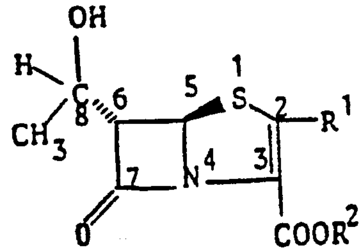

I

und deren pharmazeutisch verträgliche Salze,
worin
$R^1$ $C_3$-$C_6$-Oxocycloalkyl,
$C_3$-$C_6$-(1,1-bis-$C_1$-$C_3$-Alkyloxy)cycloalkyl,
$C_3$-$C_6$-(1,1-$C_2$-$C_3$-Alkylendioxy)cycloalkyl,
$C_3$-$C_6$-($C_1$-$C_3$-Alkylimino)cycloalkyl,
$C_3$-$C_6$-Aryliminocycloalkyl (mit Aryl gleich Phenyl, Thienyl oder Furyl),
$C_3$-$C_6$-Hydroxyiminocycloalkyl,
$C_3$-$C_6$-($C_1$-$C_3$-Alkyloxyimino)cycloalkyl,
in denen der Cycloalkylrest unsubstituiert oder ein-oder zweifach durch $C_1$-$C_3$-Alkyl, $C_1$-$C_3$-Alkyloxy, Halogen, oder durch Methylen substituiert ist
$R^2$ Wasserstoff
$C_1$-$C_5$-Alkanoyloxy-$C_1$-$C_3$-alkyl, $C_1$-$C_5$-Alkoxycarbonyloxy-$C_1$-$C_3$-alkyl, 5-Methyl-1,3-dioxolen-2-on-4-yl-methyl, Phthalidyl bedeutet,
und in denen die bevorzugte Stereochemie in Stellung 5 = R, in Stellung 6 = S und in Stellung 8 = R ist.

2. Verbindung der Formel I nach Anspruch 1, worin die folgenden Substituenten und Indices die folgende Bedeutung haben:

$R^1$

Acetale 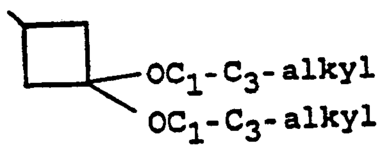

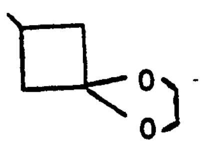 oder 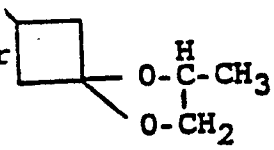

Schiffbasen 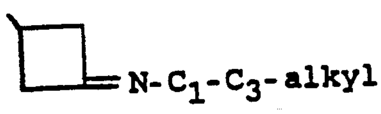 oder 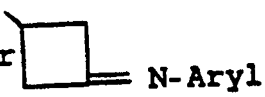

Oxime 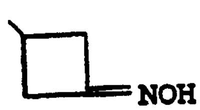 oder 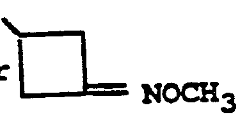 ,

worin n = 0-3 ist, und Aryl Phenyl, Thienyl, Furyl bedeutet,
$R^2$ Wasserstoff
Acetoxymethyl, Propionyloxymethyl, Isopropionyloxymethyl, N-Butyryloxymethyl, Isobutyryloxymethyl, Pivaloyloxymethyl, Isovaleryloxymethyl, 1-Acetoxyethyl, 1-Acetoxypropyl, 1-Pivaloyloxyethyl, 1-Methoxycarbonyloxyethyl, 1-Ethoxycarbonyloxyethyl, 1-Isopropoxycarbonyloxyethyl; Methoxycarbonyloxymethyl; Phthalidyl, 5-Methyl-1,3-dioxolen-2-on-4-yl-methyl.

3. Verfahren zur Herstellung von Penemderivaten der Formel I und ihrer pharmazeutisch verträglichen Salze nach Anspruch 1 dadurch gekennzeichnet, daß man

a) eine Verbindung der Formel II

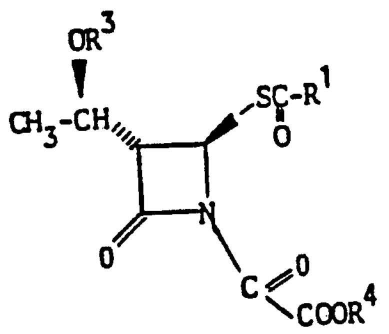

II,

worin $R^1$ die zu Formel I genannte Bedeutung hat, $R^3$ Wasserstoff oder eine Hydroxyschutzgruppe, $R^4$ eine Carboxyschutzgruppe bedeutet, mit Trialkylphosphiten umsetzt,

α) eine gegebenenfalls vorhandene Schutzgruppe $R^3$ und $R^4$ abspaltet und

ß) falls erforderlich, das erhaltene Produkt in ein pharmazeutisch verträgliches Salz überführt, oder

b) eine Verbindung der Formel III

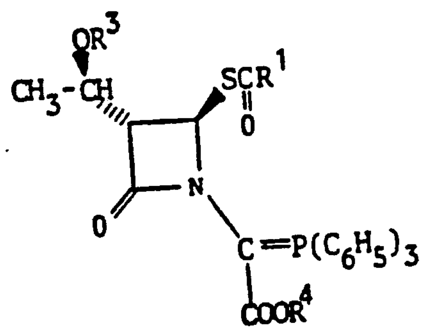

III,

worin $R^1$, $R^3$ und $R^4$ die zu Formel II genannte Bedeutung haben, durch Erhitzen cyclisiert,

α) eine gegebenenfalls vorhandene Schutzgruppe abspaltet und

ß) falls erforderlich, das erhaltene Produkt in ein pharmazeutisch verträgliches Salz überführt und

c) falls funktionelle Derivate von Ketoverbindungen erhalten werden sollen, die nach den Verfahrensvarianten a) oder b) erhaltenen Verbindungen der Formel I mit einer Ketogruppe im Rest $R^1$ in an sich bekannter Weise in Acetale, Schiffbasen oder Oxime überführt, und

d) falls in vivo spaltbare Ester erhalten werden sollen, die nach den Verfahrensvarianten a), b) oder c) erhaltenen Verbindungen der Formel I, in der $R^2$ für Wasserstoff steht, in an sich bekannter Weise in die unter $R^2$ angegebenen Ester überführt.

4. Gegen bakterielle Infektionen wirksame pharmazeutische Präparate, gekennzeichnet durch einen Gehalt an Penemderivaten der Formel I.

5. Verfahren zur Herstellung von gegen bakterielle Infektionen wirksamen pharmazeutischen Präparaten, dadurch gekennzeichnet, daß ein Penemderivat der Formel I gegebenenfalls mit pharmazeutisch üblichen Trägerstoffen oder Verdünnungsmittel in eine pharmazeutisch geeignete Verabreichungsform gebracht wird.

6. Verwendung von Penemderivaten der Formel I zur Bekämpfung bakterieller Infektionen.

7. Verwendung einer Verbindung I nach Anspruch 1 zur Herstellung eines Medikaments gegen bakterielle Infektionen.

Patentansprüche für folgende Vertragsstaaten : ES, GR

1. Verfahren zur Herstellung von Penemderivaten der Formel I

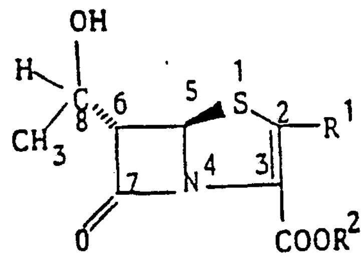

I

und von deren pharmazeutisch verträglichen Salzen,
worin
$R^1$ $C_3$-$C_6$-Oxocycloalkyl,
$C_3$-$C_6$-(1,1-bis-$C_1$-$C_3$-Alkyloxy)cycloalkyl,
$C_3$-$C_6$-(1,1-$C_2$-$C_3$-Alkylendioxy)cycloalkyl,
$C_3$-$C_6$-($C_1$-$C_3$-Alkylimino)cycloalkyl,
$C_3$-$C_6$-Aryliminocycloalkyl (mit Aryl gleich Phenyl, Thienyl oder Furyl),
$C_3$-$C_6$-Hydroxyiminocycloalkyl,
$C_3$-$C_6$-($C_1$-$C_3$-Alkyloxyimino)cycloalkyl,
in denen der Cycloalkylrest unsubstituiert oder ein-oder zweifach durch $C_1$-$C_3$-Alkyl, $C_1$-$C_3$-Alkyloxy, Halogen, oder durch Methylen substituiert ist,
$R^2$ Wasserstoff
$C_1$-$C_5$-Alkanoyloxy-$C_1$-$C_3$-alkyl, $C_1$-$C_5$-Alkoxycarbonyloxy-$C_1$-$C_3$-alkyl, 5-Methyl-1,3-dioxolen-2-on-4-yl-methyl, Phthalidyl bedeutet,
und in denen die bevorzugte Stereochemie in Stellung 5 = R, in Stellung 6 = S und in Stellung 8 = R ist, dadurch gekennzeichnet, daß man

a) eine Verbindung der Formel II

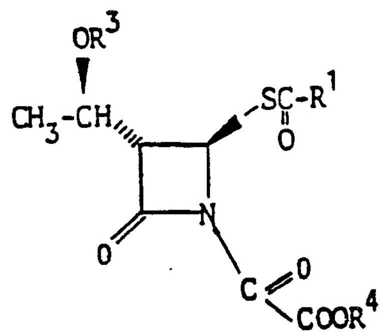

II,

worin $R^1$ die zu Formel I genannte Bedeutung hat, $R^3$ Wasserstoff oder eine Hydroxyschutzgruppe, $R^4$ eine Carboxyschutzgruppe bedeutet, mit Trialkylphosphiten umsetzt,
α) eine gegebenenfalls vorhandene Schutzgruppe $R^3$ und $R^4$ abspaltet und
β) falls erforderlich, das erhaltene Produkt in ein pharmazeutisch verträgliches Salz überführt, oder

b) eine Verbindung der Formel III

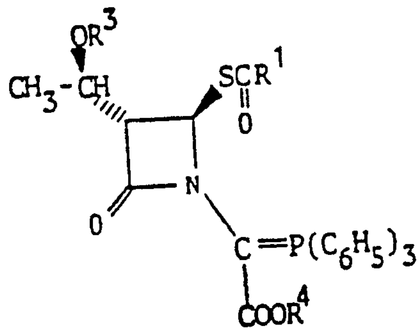

III,

worin $R^1$, $R^3$ und $R^4$ die zu Formel II genannte Bedeutung haben, durch Erhitzen cyclisiert,
α) eine gegebenenfalls vorhandene Schutzgruppe abspaltet und
β) falls erforderlich, das erhaltene Produkt in ein pharmazeutisch verträgliches Salz überführt und

c) falls funktionelle Derivate von Ketoverbindungen erhalten werden sollen, die nach den Verfahrensvarianten a) oder b) erhaltenen Verbindungen der Formel I mit einer Ketogruppe im Rest $R^2$ in an sich bekannter Weise in Acetale, Schiffbasen oder Oxime überführt, und

d) falls in vivo spaltbare Ester erhalten werden sollen, die nach den Verfahrensvarianten a), b) oder c) erhaltenen Verbindungen der Formel I, in der $R^2$ für Wasserstoff steht, in an sich bekannter Weise in die unter $R^2$ angegebenen Ester überführt.

2. Verfahren nach Anspruch 1, dadurch gekennzeichnet, daß die folgenden Substituenten und Indices die folgende Bedeutung haben:

$R^1$

Acetale 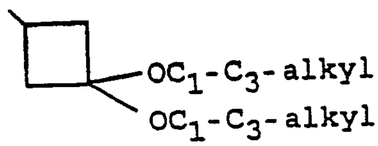

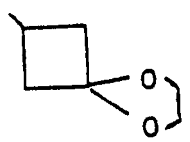 oder 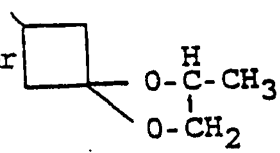

Schiffbasen 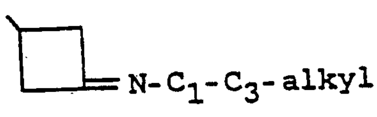 oder 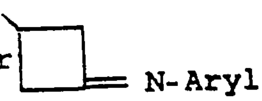

Oxime 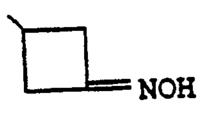 oder 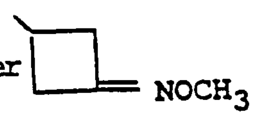 ,

worin n = 0-3 ist, und Aryl Phenyl, Thienyl, Furyl bedeutet,
$R^2$ Wasserstoff
Acetoxymethyl, Propionyloxymethyl, Isopropionyloxymethyl, N-Butyryloxymethyl, Isobutyryloxymethyl, Pivaloyloxymethyl, Isovaleryloxymethyl, 1-Acetoxyethyl, 1-Acetoxypropyl, 1-Pivaloyloxyethyl, 1-Methoxycarbonyloxyethyl, 1-Ethoxycarbonyloxyethyl, 1-Isopropoxycarbonyloxyethyl; Methoxycarbonyloxymethyl; Phthalidyl, 5-Methyl-1,3-dioxolen-2-on-4-yl-methyl.

3. Verfahren zur Herstellung von gegen bakterielle Infektionen wirksamen pharmazeutischen Präparaten, dadurch gekennzeichnet, daß ein Penemderivat der Formel I gegebenenfalls mit pharmazeutisch üblichen Trägerstoffen oder Verdünnungsmittel in eine pharmazeutisch geeignete Verabreichungsform gebracht wird.

4. Verwendung von Penemderivaten der Formel I zur Bekämpfung bakterieller Infektionen.

5. Verwendung einer Verbindung I nach Anspruch 1 zur Herstellung eines Medikaments gegen

bakterielle Infektionen.